# FASCICULE DE BREVET EUROPEEN

(11) **EP 3 478 357 B1**
(45) Date de publication et mention de la délivrance du brevet: **25.08.2021**
(21) Numéro de dépôt: 18723577.5
(22) Date de dépôt: 26.04.2018
(51) Int. Cl.: A61M 35/00, A61M 37/00, A61B 17/20

(54) **APPLICATEUR D'UN PRODUIT DE TRAITEMENT COSMÉTIQUE DE LA PEAU OU DES LÈVRES**
APPLIKATOR FÜR EIN KOSMETISCHES PRODUKT ZUR BEHANDLUNG DER HAUT ODER DER LIPPEN
APPLICATOR FOR A COSMETIC TREATMENT PRODUCT FOR THE SKIN OR LIPS

(30) Priorité: 28.04.2017 FR 1753774
(43) Date de publication de la demande: 08.05.2019
(73) Titulaire: Paradis, Line, Dubai (AE)
(72) Inventeur: Paradis, Line, Dubai (AE)
(74) Mandataire: Cabinet Beau de Loménie
(86) Numéro de dépôt international: PCT/FR2018/051058
(87) Numéro de publication internationale: WO 2018/197815

(56) Documents cités:
- EP-A1- 2 011 539
- US-A1- 2013 197 560

## Description

### Arrière-plan de l'invention

Il existe aujourd'hui plusieurs types de méthodes qui peuvent être mises en œuvre afin d'effacer un tatouage de la peau.

On peut, en particulier, utiliser des méthodes chimiques dans lesquelles une composition liquide de détatouage est injectée, à travers la peau, dans le derme au niveau du tatouage. Cette technique utilise un système assez similaire à celui utilisé pour le tatouage, comprenant une pluralité d'aiguilles aptes à perforer la peau pour y injecter la composition de détatouage. Les compositions de détatouage connues permettent de dissoudre les pigments du tatouage et provoquent leur rejet à la surface de la peau.

Toutefois ces techniques de détatouage présentent un caractère invasif dans la mesure où elles nécessitent de perforer la peau durant le traitement. De telles techniques produisent un inconfort pour le sujet subissant le traitement de détatouage et peuvent conduire à l'apparition ultérieure de cicatrices inesthétiques.

Ce problème lié à la perforation de la surface traitée par le dispositif d'application peut aussi se rencontrer dans le cadre d'autres traitements cosmétiques, comme par exemple dans le cas du maquillage permanent.

On connait en outre US 2013/0197560 qui divulgue des arrangements d'aiguilles utiles pour le détatouage. On connait encore EP 2 011 539 qui divulgue des aiguilles de tatouage.

Il existe donc un besoin pour fournir un dispositif permettant de réaliser un traitement cosmétique efficace de la peau ou des lèvres, tout en limitant, voire en évitant, la perforation de la surface traitée.

### Objet et résumé de l'invention

A cet effet, l'invention propose, selon un premier aspect, un applicateur selon la revendication 1.

Pour des raisons de concision, l'expression « section transversale par rapport à l'axe longitudinal » sera, sauf mention contraire, désignée dans la suite par « section transversale ».

L'applicateur selon l'invention permet de réaliser un traitement cosmétique efficace tout en réduisant le risque d'une perforation de la surface de la peau ou des lèvres traitée. En particulier, l'invention est remarquable du fait de la présence du premier ensemble d'aiguilles disposées en carré. Cette disposition procure une répartition équilibrée de la pression appliquée par les aiguilles sur la surface traitée, ce qui réduit le risque de perforation de cette surface tout en permettant de réaliser un traitement cosmétique efficace.

Le faisceau d'aiguilles comprend en outre un deuxième ensemble d'aiguilles situé autour du premier ensemble d'aiguilles et définissant un contour du faisceau d'aiguilles présentant une forme arrondie en section transversale par rapport à l'axe longitudinal.

Afin de minimiser le risque de perforation, l'utilisateur impose préférentiellement, lors de l'utilisation, un angle proche de 90° entre l'axe longitudinal le long duquel se déplacent les aiguilles et la surface traitée. Toutefois, selon la dextérité de l'utilisateur, le maintien de cet angle peut être plus ou moins respecté durant le traitement. La mise en œuvre d'un tel faisceau d'aiguilles à contour arrondi permet de limiter le risque de perforation de la surface, et ce même si l'utilisateur ne maintient pas l'angle précité proche de la valeur de 90°. Un tel faisceau permet ainsi d'introduire une grande tolérance en termes d'inclinaison possible de l'axe longitudinal par rapport à la surface durant l'application. Le deuxième ensemble d'aiguilles permet ainsi de « protéger » la surface traitée des sommets pointus du carré même en cas d'inclinaison notable de l'axe longitudinal par rapport à la surface traitée.

En particulier, le carré défini par le premier ensemble d'aiguilles présente une pluralité de sommets, et chaque sommet est situé entre deux aiguilles consécutives du deuxième ensemble d'aiguilles.

Comme il sera décrit plus en détails dans la suite, le contour de forme arrondie peut présenter, en section transversale, une forme de carré à sommets arrondis ou bien une forme circulaire.

Dans un exemple de réalisation, les aiguilles formant le faisceau d'aiguilles sont des aiguilles à pointe courte.

Par « aiguille à pointe courte », il faut comprendre une aiguille ayant une pointe qui s'étend sur une longueur inférieure ou égale à 2 mm.

Dans un exemple de réalisation, les aiguilles formant le faisceau d'aiguilles présentent un diamètre compris entre 0,25 mm et 0,40 mm.

Par « diamètre », on entend, sauf mention contraire, la plus grande dimension transversale mesurée perpendiculairement à l'axe X longitudinal.

Ce diamètre peut par exemple être compris entre 0,25 mm et 0,35 mm ou entre 0,30 mm et 0,40 mm. En particulier, ce diamètre peut être sensiblement égal à 0,30 mm ou à 0,35 mm.

Dans un exemple de réalisation, l'applicateur est sous la forme d'une pièce à main.

Dans un exemple de réalisation, les aiguilles du faisceau d'aiguilles sont aimantées.

Une telle caractéristique est avantageuse dans la mesure où l'aimantation des aiguilles permet d'encore mieux attirer l'oxyde de fer des pigments du tatouage en dehors de la peau ou des lèvres et donc d'améliorer davantage encore l'efficacité de détatouage.

Dans un exemple de réalisation, les aiguilles du faisceau d'aiguilles sont en acier inoxydable et présentent une teneur massique en nickel inférieure ou égale à 9%.

L'invention vise également, selon un deuxième aspect, un procédé de traitement cosmétique mettant en œuvre un applicateur tel que décrit plus haut, le procédé comprenant au moins l'étape suivante :
- application sur une surface de la peau ou des lèvres d'un produit de traitement cosmétique présent sur le faisceau d'aiguilles, le faisceau d'aiguilles étant animé d'un mouvement de va et vient le long de l'axe longitudinal durant l'application.

Dans un exemple de réalisation, l'axe longitudinal forme un angle compris entre 45° et 135° par rapport à la surface traitée durant l'application.

Les applicateurs ayant un faisceau d'aiguilles à contour arrondi évoqués plus haut peuvent avantageusement être utilisés sur toute cette plage d'angle durant le traitement.

L'applicateur peut être utilisé dans des applications cosmétiques diverses. On peut en particulier appliquer à l'aide de cet applicateur un produit cosmétique destiné à migrer au travers de la surface traitée. On peut dans ce cas réaliser un traitement de dermopigmentation, d'introduction d'un composé dans la peau ou les lèvres ou d'élimination d'un composé présent dans la peau ou les lèvres, comme un traitement de détatouage. Ainsi, le procédé de traitement peut être un procédé de détatouage de la peau ou des lèvres. En variante, le procédé de traitement peut être un procédé de maquillage permanent de la peau ou des lèvres. En variante encore, le procédé peut être un procédé d'introduction d'acide hyaluronique dans la peau ou dans les lèvres. En variante encore, le procédé peut être un procédé d'élimination de l'acide hyaluronique introduit au préalable dans la peau ou dans les lèvres.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- la figure 1 illustre, de manière schématique et partielle, un exemple d'applicateur selon l'invention,
- la figure 1A représente, de manière schématique, une aiguille à pointe courte, prise isolément, faisant partie du faisceau d'aiguilles utilisé dans l'applicateur de la figure 1,
- la figure 2 est une vue, en section transversale, du faisceau d'aiguilles de l'applicateur de la figure 1,
- la figure 3 est une vue, en section transversale, d'une variante de faisceau d'aiguilles utilisable dans le cadre de l'invention,
- la figure 4 est une vue, en section transversale, d'un faisceau d'aiguilles ne faisant pas partie de l'invention,
- la figure 5 est une vue, en section transversale, d'un faisceau d'aiguilles ne faisant pas partie de l'invention,
- la figure 6 illustre, de manière schématique et partielle, la réalisation d'un exemple de procédé de traitement cosmétique selon l'invention, et
- les figures 7A à 7B sont des photographies montrant le résultat de détatouage obtenu à l'aide d'un applicateur selon l'invention ayant un faisceau à 88 aiguilles,
- les figures 8A et 8B sont des photographies montrant le résultat de maquillage permanent obtenu à l'aide d'un applicateur selon l'invention ayant un faisceau à 88 aiguilles, et
- la figure 9 est une photographie montrant le résultat obtenu lorsque le faisceau à 52 aiguilles divulgué à la figure 3c du document US 2013/0197560 est mis en œuvre.

### Description détaillée de modes de réalisation

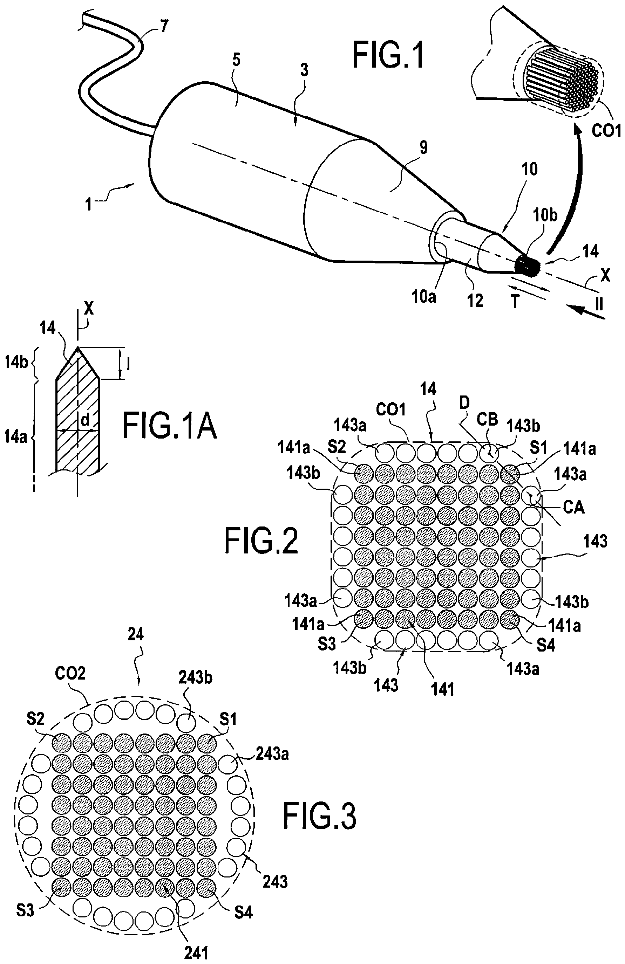

On a représenté à la figure 1 un exemple d'applicateur 1 selon l'invention. L'applicateur 1 se présente sous la forme d'une pièce à main. L'applicateur 1 comprend un corps 3 présentant une partie proximale 5 dans laquelle un dispositif d'entraînement en translation est logé. Le dispositif d'entraînement est configuré pour imposer un mouvement de va et vient en translation au faisceau d'aiguilles 14. Le dispositif d'entraînement constitue un dispositif motorisé qui n'est pas original en soi. Le dispositif d'entraînement est relié à un générateur électrique (non représenté) par l'intermédiaire du câble électrique 7. Le corps 3 présente en outre une partie distale 9 formant partie de préhension. A titre d'exemple de corps 3 renfermant un dispositif d'entraînement utilisable dans le cadre de l'invention, on peut citer le dispositif commercial « Dermo Power Pen » (Linda Paradis).

L'applicateur 1 comprend, en outre, un embout 10 relié au corps 3. Cet embout 10 comprend une enveloppe 12 entourant le faisceau d'aiguilles 14. Le faisceau d'aiguilles 14 s'étend à l'intérieur de l'enveloppe 12. Les aiguilles formant le faisceau d'aiguilles 14 sont solidaires entre elles et sont ainsi maintenues dans une position prédéterminée. Le faisceau 14 s'étend le long d'un axe X longitudinal. L'ensemble constitué par l'enveloppe 12 et le faisceau d'aiguilles 14 se présente ici sous la forme d'une cartouche qui est reliée au corps 3. L'enveloppe 12 définit ainsi une portion de fixation 10a de l'embout 10 qui est insérée dans un logement ménagé dans la partie distale 9 du corps 3 afin de fixer l'embout 10 au corps 3.

Le faisceau d'aiguilles 14 forme la partie d'application de l'applicateur 1. Les aiguilles 14 sont ainsi destinées à être mises en contact avec le produit cosmétique afin de réaliser son application sur la surface traitée. La partie distale 10b de l'embout 10 est définie par le faisceau d'aiguilles 14. Le faisceau d'aiguilles 14 dépasse de l'enveloppe 12.

Les aiguilles 14 sont, bien entendu, stériles avant utilisation de l'applicateur 1. Les aiguilles 14 sont à usage unique. Les aiguilles 14 peuvent être formées d'un matériau métallique, comme de l'acier inoxydable. On peut par exemple utiliser des aiguilles 14 en acier inoxydable appartenant aux séries 200 ou 400 et ayant une teneur massique en nickel inférieure ou égale à 9%, par exemple comprise entre 3% et 9%. Le diamètre d de chacune des aiguilles 14 peut être compris entre 0,25 mm et 0,40 mm, et par exemple être sensiblement égal à 0,3 mm ou à 0,35 mm. Comme illustré sur la figure 1A, les aiguilles 14 peuvent comprendre une portion de diamètre constant 14a prolongée par une portion effilée 14b de diamètre décroissant. La portion effilée 14b définit la pointe de l'aiguille, cette portion 14b est destinée à venir en contact avec la surface à traiter. On peut utiliser des aiguilles 14 à pointe courte (aiguilles connues en soi, communément désignées par l'expression anglaise « short-taper needles »). De telles aiguilles 14 présentent une pointe 14b ayant une longueur l inférieure ou égale à 2 mm.

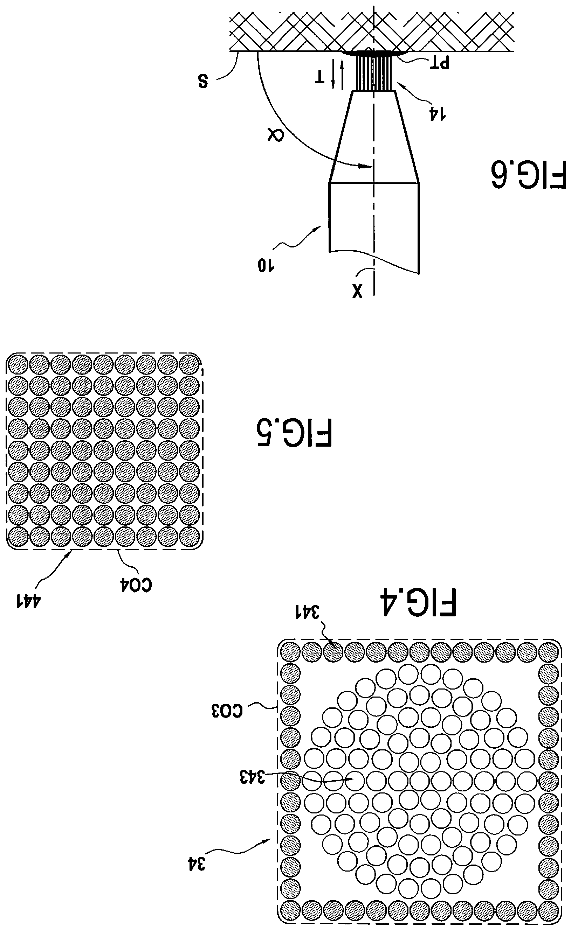

Lors de l'actionnement du dispositif d'entraînement, les aiguilles 14 sont animées d'un mouvement de va et vient le long de l'axe X. Ce mouvement de va et vient est matérialisé par la double flèche T aux figures 1 et 5. Ce mouvement de va et vient constitue un mouvement de translation alternatif en avant et en arrière le long de l'axe X. Lorsque le dispositif d'entraînement est actionné, le faisceau d'aiguilles 14 se déplace en bloc avec un mouvement de va et vient le long de l'axe X.

Comme évoqué plus haut, l'invention est remarquable en ce que le faisceau d'aiguilles comprend un premier ensemble d'aiguilles ayant une répartition en carré en section transversale ainsi qu'un deuxième ensemble d'aiguilles situé autour et définissant un contour du faisceau d'aiguilles présentant une forme arrondie en section transversale. La mise en œuvre d'un tel premier ensemble d'aiguilles limitant le risque de perforation de la surface traitée. Diverses géométries possibles pour le faisceau d'aiguilles vont à présent être décrites en lien avec les figures 2 à 5 (les figures 4 et 5 sont hors invention).

La figure 2 représente le faisceau d'aiguilles 14 utilisé dans l'applicateur de la figure 1 vu en section transversale par rapport à l'axe X. La section transversale peut être prise perpendiculairement à l'axe X.

Le faisceau d'aiguilles 14 comprend un premier ensemble d'aiguilles 141 qui forme un carré lorsque le faisceau 14 est observé en section transversale. Ce carré présente une pluralité de sommets S1-S4. Les aiguilles formant le premier ensemble d'aiguilles 141 sont en contact les unes avec les autres.

Dans l'exemple de la figure 2, Le faisceau d'aiguilles 14 comprend, en outre, un deuxième ensemble d'aiguilles 143 situé autour du premier ensemble d'aiguilles 141. Chaque sommet S1-S4 est situé entre deux aiguilles consécutives 143a et 143b du deuxième ensemble d'aiguilles 143. L'expression « deux aiguilles consécutives du deuxième ensemble d'aiguilles » désigne deux aiguilles du deuxième ensemble d'aiguilles 143 qui sont consécutives lorsque l'on se déplace autour du premier ensemble d'aiguilles 141. Ainsi, chaque aiguille 141a du premier ensemble située au niveau d'un sommet S1-S4 est située entre deux aiguilles consécutives 143a et 143b du deuxième ensemble. Lorsque le faisceau 14 est observé en section transversale, chaque aiguille 141a est située sur la droite D reliant les centres CA et CB des deux aiguilles consécutives 143a et 143b situées de part et d'autre de l'aiguille 141a considérée.

Dans l'exemple illustré aux figures 1 et 2, le contour CO1 du faisceau 14 est défini par les aiguilles du deuxième ensemble d'aiguilles 143. Le contour CO1 longe les aiguilles du deuxième ensemble 143. Le contour CO1 constitue une surface de forme tubulaire. Le contour CO1 délimite le faisceau d'aiguilles 14. Le contour CO1 constitue la surface externe du faisceau d'aiguilles 14. Le contour CO1 s'étend le long de l'axe X. Le faisceau d'aiguilles 14 est présent à l'intérieur du contour CO1.

Le contour CO1 présente ici, en section transversale, une forme de carré à sommets arrondis. En effet, la présence des aiguilles du deuxième ensemble 143 arrondit la forme des sommets S1-S4 du carré défini par le premier ensemble d'aiguilles 141. Comme évoqué plus haut, la présence du deuxième ensemble 143 permet, durant l'utilisation, d'introduire une grande tolérance en termes d'inclinaison possible pour le faisceau 14 par rapport à la surface traitée, tout en limitant le risque de perforation de celle-ci. Les aiguilles du deuxième ensemble 143 permettent ainsi de « protéger » la surface traitée des sommets pointus S1-S4 en cas d'une forte inclinaison du faisceau durant l'utilisation.

Dans la géométrie particulière illustrée à la figure 2, les aiguilles du deuxième ensemble 143 sont situées le long de chaque côté du carré formé par les aiguilles du premier ensemble 141. Une unique rangée d'aiguilles du deuxième ensemble 143 est présente le long de chaque côté du carré formé par les aiguilles du premier ensemble 141. Dans chacune de ces rangées, les aiguilles 143 sont alignées parallèlement au côté du carré situé en regard de la rangée considérée.

L'exemple de faisceau 14 illustré ici comprend 88 aiguilles réparties en 64 aiguilles (carré 8x8) pour le premier ensemble 141 et 24 aiguilles (6x4) pour le deuxième ensemble 143. En variante, on pourrait par exemple utiliser un faisceau ayant la même géométrie mais comportant 132 aiguilles (10x10 + 8x4). Ces valeurs de 88 et 132 aiguilles permettent d'obtenir une efficacité de traitement maximale pour cette géométrie. La mise en œuvre de faisceaux d'aiguilles à 88 aiguilles (8x8 + 6x4) ou 132 aiguilles (10x10 + 8x4) permet d'éviter de perforer la surface traitée durant le traitement cosmétique.

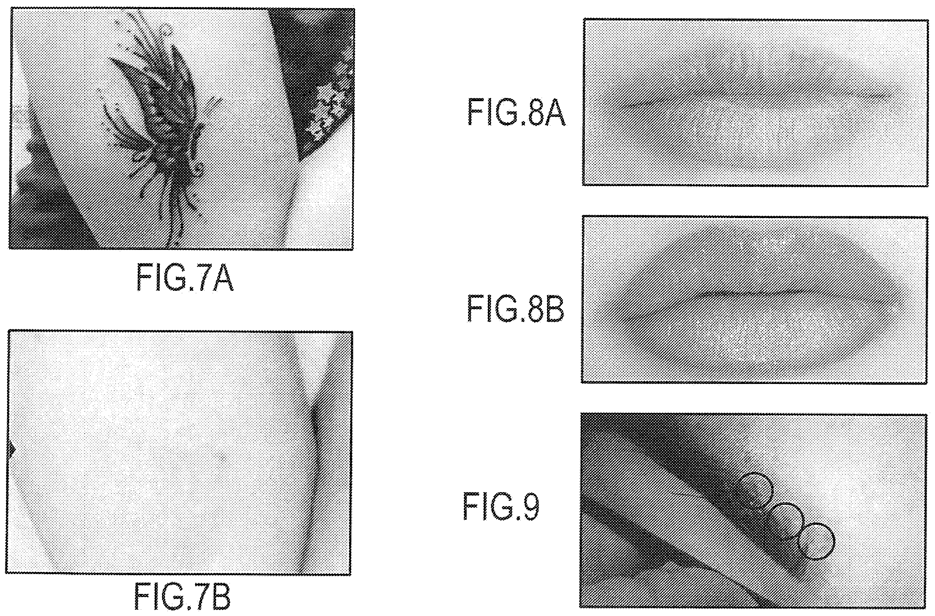

Les figures 7A et 7B sont des photographies montrant le résultat de détatouage obtenu à l'aide d'un applicateur selon l'invention ayant un faisceau à 88 aiguilles (8x8 aiguilles pour premier ensemble et 6x4 aiguilles pour le deuxième ensemble). On constate que ce traitement ne conduit à aucune perforation de la peau.

Les figures 8A et 8B sont des photographies montrant le résultat de maquillage permanent obtenu à l'aide d'un applicateur selon l'invention ayant ce même faisceau à 88 aiguilles. On constate que ce traitement ne conduit à aucune perforation des lèvres.

La mise en œuvre d'un faisceau à 132 aiguilles selon l'invention fournit aussi un résultat de traitement cosmétique satisfaisant sans perforation de la surface traitée.

En revanche, la figure 9 montre qu'une perforation de la peau est obtenue lorsqu'un faisceau à 52 aiguilles tel divulgué à la figure 3c du document US 2013/0197560 est mis en œuvre. Les zones perforées de la peau sont entourées sur la figure 9.

Ainsi, en comparaison avec le faisceau d'aiguilles divulgué dans le document US 2013/0197560, l'emploi de faisceaux à 88 ou 132 aiguilles permet avantageusement d'éviter de perforer la surface traitée.

Par ailleurs et comme indiqué plus haut, les aiguilles du faisceau d'aiguilles, et en particulier des premier 141 et deuxième 143 ensembles d'aiguilles, peuvent être aimantées.

Afin d'aimanter les aiguilles, on peut utiliser un aimant permanent ayant un champ d'induction magnétique supérieur à 100 Gauss, par exemple compris entre 100 Gauss et 6000 Gauss.

Bien entendu on ne sort pas du cadre de l'invention si les aiguilles mises en œuvre ne sont pas aimantées.

La figure 3 illustre une variante de faisceau d'aiguilles 24 dont le contour présente aussi une forme arrondie en section transversale.

Le faisceau d'aiguilles 24 comprend un premier ensemble d'aiguilles 241 ayant une répartition en carré en section transversale, ainsi qu'un deuxième ensemble d'aiguilles 243 situé autour du premier ensemble 241.

Le deuxième ensemble d'aiguilles 243 confère au faisceau d'aiguilles 24 un contour CO2 de forme arrondie en section transversale. A la différence de l'exemple de la figure 2, le contour CO2 a ici une forme circulaire en section transversale, et pas une forme de carré à sommets arrondis.

De la même manière que dans l'exemple de la figure 2, chaque sommet S1 est situé entre deux aiguilles consécutives 243a et 243b du deuxième ensemble 243. Le faisceau d'aiguilles 24 illustré à la figure 3 présente, en section transversale, une forme de carré inscrit dans un cercle.

De la même manière que pour le faisceau 14 de la figure 2, le faisceau 24 peut avantageusement être formé de 88 aiguilles (premier ensemble 241 : 64 aiguilles, et deuxième ensemble 243 : 24 aiguilles), ou être formé de 132 aiguilles (premier ensemble 241 : 100 aiguilles, et deuxième ensemble 243 : 32 aiguilles).

Les exemples de faisceaux 14 et 24 qui viennent d'être décrits en lien avec les figures 2 et 3 présentent chacun, en section transversale, un contour de forme arrondie. Il est également décrit des faisceaux ayant en section transversale un contour ayant une forme différente qui vont à présent être décrits en lien avec les figures 4 et 5 sans toutefois faire partie de l'invention.

Dans l'exemple de la figure 4, le faisceau 34 comprend un premier ensemble d'aiguilles 341 ayant une répartition en carré en section transversale. Le faisceau 34 comprend, en outre, un ensemble d'aiguilles additionnel 343, le premier ensemble d'aiguilles 341 étant situé autour de l'ensemble d'aiguilles 343 additionnel. L'ensemble additionnel 343 a ici une répartition circulaire en section transversale.

Dans cet exemple, le contour CO3 du faisceau 34 est défini par le premier ensemble d'aiguilles 341 et présente une forme carrée en section transversale. Le contour CO3 longe les aiguilles du premier ensemble 341.

Le faisceau d'aiguilles 34 illustré à la figure 4 présente, en section transversale, une forme de cercle inscrit dans un carré.

La figure 5 illustre le cas où le faisceau d'aiguilles 441 est uniquement formé par le premier ensemble d'aiguilles 441. Le contour C04 du faisceau 441 présente ainsi, en section transversale, une forme carrée. Dans ce cas, le faisceau 441 peut comporter 81 (carré 9×9) ou 100 (carré 10×10) aiguilles.

Diverses géométries possibles pour le faisceau d'aiguilles, faisant partie de l'invention ou non, viennent d'être décrites. La mise en œuvre d'un exemple de traitement cosmétique selon l'invention va maintenant être décrite en lien avec la figure 6.

La figure 6 illustre la mise en œuvre d'un traitement cosmétique d'une surface S de la peau ou des lèvres à l'aide d'un applicateur 1 selon l'invention. La surface S peut, en particulier, être une zone du visage ou du corps.

Dans un premier temps, un produit PT de traitement cosmétique est appliqué sur le faisceau d'aiguilles 14. Ce produit PT peut être à l'état liquide. Le produit PT peut être configuré pour migrer au travers de la surface S. La pression alternative produite par le mouvement de va et vient du faisceau d'aiguilles 14 participe à faciliter cette migration. Le produit PT peut permettre de réaliser le traitement cosmétique au niveau du derme. L'applicateur 1 peut permettre de réaliser un traitement de dermopigmentation de la peau ou des lèvres, un traitement d'introduction d'acide hyaluronique dans la peau ou les lèvres, un traitement de détatouage de la peau ou des lèvres ou un procédé d'élimination d'acide hyaluronique présent dans la peau ou les lèvres.

Dans le cas du détatouage, le produit PT est une composition liquide de détatouage apte à migrer au travers de la surface S. Une telle composition liquide permet d'extraire l'encre de tatouage à l'extérieur de la surface S afin de réaliser le détatouage. A titre d'exemple de composition de détatouage utilisable, on peut citer la composition commerciale « Tattoo Remoov » ™ (Linda Paradis).

Dans le cas du traitement de dermopigmentation, on peut réaliser un maquillage permanent de la peau ou des lèvres en utilisant un produit PT de maquillage permanent apte à migrer au travers de la surface S. Un tel produit peut être à base de pigments inorganiques, et constitue aussi un produit connu en soi.

Dans le cas d'un traitement d'élimination de l'acide hyaluronique, on peut appliquer un produit PT constitué par une hyaluronidase à l'aide de l'applicateur.

Afin de réaliser l'application, les aiguilles 14 imprégnées du produit PT sont animées d'un mouvement de va et vient le long de l'axe X. Le faisceau 14 se déplace transversalement par rapport à la surface traitée durant l'application. Le faisceau 14 d'aiguilles a un effet de piston qui facilite la pénétration du produit. Dans l'exemple de la figure 6, l'axe X est maintenu durant l'application à un angle α sensiblement égal à 90° par rapport à la surface S. Cette configuration permet de limiter au maximum le risque de perforation de la surface S. On peut toutefois obtenir des résultats satisfaisants sur toute l'amplitude 45°-135° pour l'angle α si l'on utilise un faisceau à contour arrondi, comme le faisceau 14 de la figure 2 ou le faisceau 24 de la figure 3.

L'expression « compris(e) entre ... et ... » doit se comprendre comme incluant les bornes.

## Revendications

1. Applicateur (1) d'un produit de traitement (PT) cosmétique de la peau ou des lèvres, l'applicateur comprenant au moins :
- une partie d'application comprenant un faisceau d'aiguilles (14 ; 24) destiné à appliquer le produit de traitement cosmétique et s'étendant le long d'un axe (X) longitudinal, le faisceau d'aiguilles comprenant au moins un premier ensemble d'aiguilles (141 ; 241) ayant une répartition en carré en section transversale par rapport à l'axe longitudinal, et
- un dispositif d'entraînement en translation relié à la partie d'application et configuré pour imposer au faisceau d'aiguilles (14 ; 24) un mouvement (T) de va et vient le long de l'axe (X) longitudinal,
l'applicateur étant **caractérisé en ce que** le faisceau d'aiguilles comprend en outre un deuxième ensemble d'aiguilles (143; 243) situé autour du premier ensemble d'aiguilles et définissant un contour (CO1 ; CO2) du faisceau d'aiguilles présentant une forme arrondie en section transversale par rapport à l'axe longitudinal,
et **en ce que** le faisceau d'aiguilles comprend :
- 88 aiguilles réparties en 64 aiguilles pour le premier ensemble d'aiguilles et 24 aiguilles pour le deuxième ensemble d'aiguilles, ou
- 132 aiguilles réparties en 100 aiguilles pour le premier ensemble d'aiguilles et 32 aiguilles pour le deuxième ensemble d'aiguilles.

2. Applicateur (1) selon la revendication 1, dans lequel le carré défini par le premier ensemble d'aiguilles (141 ; 241) présente une pluralité de sommets (S1-Sn), et dans lequel chaque sommet est situé entre deux aiguilles consécutives (143a ; 143b ; 243a ; 243b) du deuxième ensemble d'aiguilles.

3. Applicateur (1) selon l'une quelconque des revendications 1 ou 2, dans lequel les aiguilles formant le faisceau d'aiguilles (14 ; 24) sont des aiguilles à pointe courte ayant une pointe qui s'étend sur une longueur inférieure ou égale à 2 mm.

4. Applicateur (1) selon l'une quelconque des revendications 1 à 3, dans lequel les aiguilles (14 ; 24) formant le faisceau d'aiguilles présentent un diamètre (d) compris entre 0,25 mm et 0,40 mm.

5. Applicateur (1) selon l'une quelconque des revendications 1 à 4, dans lequel l'applicateur est sous la forme d'une pièce à main.

6. Applicateur (1) selon l'une quelconque des revendications 1 à 5, dans lequel les aiguilles du faisceau d'aiguilles sont aimantées.

7. Procédé de traitement cosmétique mettant en œuvre un applicateur (1) selon l'une quelconque des revendications 1 à 6, le procédé comprenant au moins l'étape suivante :
- application sur une surface (S) de la peau ou des lèvres d'un produit de traitement (PT) cosmétique présent sur le faisceau d'aiguilles (14 ; 24), le faisceau d'aiguilles étant animé d'un mouvement (T) de va et vient le long de l'axe (X) longitudinal durant l'application.

8. Procédé selon la revendication 7, dans lequel le procédé est un procédé de détatouage de la peau ou des lèvres.

9. Procédé selon la revendication 7, dans lequel le procédé est un procédé de maquillage permanent de la peau ou des lèvres.

10. Procédé selon la revendication 7, dans lequel le procédé est un procédé d'introduction d'acide hyaluronique dans la peau ou dans les lèvres.

11. Procédé selon la revendication 7, dans lequel le procédé est un procédé d'élimination de l'acide hyaluronique introduit au préalable dans la peau ou dans les lèvres.

## Patentansprüche

1. Applikator (1) eines kosmetischen Behandlungsprodukts (PT) der Haut oder der Lippen, wobei der Applikator mindestens umfasst:
- einen Applikationsteil, der ein Nadelbündel (14; 24) umfasst, das bestimmt ist, das kosmetische Behandlungsprodukt aufzutragen und sich entlang einer Längsachse (X) erstreckt, wobei das Nadelbündel mindestens eine erste Nadeleinheit (141; 241) mit einer quadratischen Verteilung im Querschnitt in Bezug auf die Längsachse umfasst, und
- eine Translations-Antriebsvorrichtung, die mit dem Applikationsteil verbunden und ausgelegt ist, um das Nadelbündel (14; 24) einer Hin- und Her-Bewegung (T) entlang der Längsachse (X) zu unterziehen,
wobei der Applikator **dadurch gekennzeichnet ist, dass** das Nadelbündel ferner eine zweite Nadeleinheit (143; 243) umfasst, die sich um die erste Nadeleinheit herum befindet und eine Kontur (CO1; CO2) des Nadelbündels definiert, die eine abgerundete Form im Querschnitt in Bezug auf die Längsachse aufweist,
und dass das Nadelbündel umfasst:
- 88 Nadeln, die in 64 Nadeln für die erste Nadeleinheit und 24 Nadeln für die zweite Nadeleinheit aufgeteilt sind, oder
- 132 Nadeln, die in 100 Nadeln für die erste Nadeleinheit und 32 Nadeln für die zweite Nadeleinheit aufgeteilt sind.

2. Applikator (1) nach Anspruch 1, wobei das von der ersten Nadeleinheit (141; 241) definierte Quadrat eine Vielzahl von Erhebungen (S1-Sn) aufweist und wobei sich jede Erhebung zwischen zwei aufeinanderfolgenden Nadeln (143a; 143b; 243a; 243b) der zweiten Nadeleinheit befindet.

3. Applikator (1) nach einem der Ansprüche 1 oder 2, wobei die Nadeln, die das Nadelbündel (14; 24) bilden, Nadeln mit kurzer Spitze sind mit einer Spitze, die sich über eine Länge von unter oder gleich 2 mm erstreckt.

4. Applikator (1) nach einem der Ansprüche 1 bis 3, wobei die Nadeln (14; 24), die das Nadelbündel bilden, einen Durchmesser (d) aufweisen, der zwischen 0,25 mm und 0,40 mm liegt.

5. Applikator (1) nach einem der Ansprüche 1 bis 4, wobei der Applikator in Form eines Handstücks vorliegt.

6. Applikator (1) nach einem der Ansprüche 1 bis 5, wobei die Nadeln des Nadelbündels magnetisiert sind.

7. Verfahren zur kosmetischen Behandlung, das einen Applikator (1) nach einem der Ansprüche 1 bis 6 verwendet, wobei das Verfahren mindestens den folgenden Schritt aufweist:
- Auftragen, auf eine Fläche (S) der Haut oder der Lippen, eines kosmetischen Behandlungsprodukts (PT), das auf dem Nadelbündel (14; 24) vorhanden ist, wobei das Nadelbündel während der Applikation in einer Hin- und Her-Bewegung (T) entlang der Längsachse (X) bewegt wird.

8. Verfahren nach Anspruch 7, wobei das Verfahren ein Verfahren zum Entfernen von Tätowierungen der Haut oder der Lippen ist.

9. Verfahren nach Anspruch 7, wobei das Verfahren ein Permanent-Make-up-Verfahren der Haut oder der Lippen ist.

10. Verfahren nach Anspruch 7, wobei das Verfahren ein Verfahren zum Einschleusen von Hyaluronsäure in die Haut oder in die Lippen ist.

11. Verfahren nach Anspruch 7, wobei das Verfahren ein Verfahren zum Entfernen von zuvor in die Haut oder in die Lippen eingeschleuster Hyaluronsäure ist.

## Claims

1. An applicator (1) for a cosmetic treatment product (PT) for the skin or lips, the applicator comprising at least:
- an application portion comprising a bundle of needles (14; 24) for applying the cosmetic treatment product and extending along a longitudinal axis (X), the bundle of needles comprising at least a first set of needles (141; 241) being arranged in a square in cross-section relative to the longitudinal axis, and
- a linear drive device connected to the application portion and configured to drive the bundle of needles (14; 24) with reciprocating motion (T) along the longitudinal axis (X),
the applicator being **characterized in that** the bundle of needles further comprises a second set of needles (143; 243) situated around the first set of needles and defining an outline (CO1; CO2) for the bundle of needles that presents a rounded shape in cross-section relative to the longitudinal axis
and **in that** the bundle of needles comprises:
- 88 needles distributed as 64 needles in the first set of needles and 24 needles in the second set of needles, or
- 132 needles distributed as 100 needles in the first set of needles and 32 needles in the second set of needles.

2. The applicator (1) as claimed in claim 1, wherein the square defined by the first set of needles (141; 241) presents a plurality of corners (S1-Sn), and wherein each corner is situated between two consecutive needles (143a; 143b; 243a; 243b) of the second set of needles.

3. The applicator (1) as claimed in any one of claims 1 or 2, wherein the needles forming the bundle of needles (14; 24) are short-taper needles having a length that is 2 mm or less.

4. The applicator (1) as claimed in any one of claims 1 to 3, wherein the needles (14; 24) forming the bundle of needles present a diameter (d) lying in the range 0.25 mm to 0.40 mm.

5. The applicator (1) as claimed in any one of claims 1 to 4, wherein the applicator is in the form of a handpiece.

6. The applicator (1) as claimed in any one of claims 1 to 5, wherein the needles of the bundle of needles are magnetized.

7. A cosmetic treatment method implementing an applicator (1) as claimed in any one of claims 1 to 6, the method comprising at least the following step:
- applying to a surface (S) of the skin or the lips a cosmetic treatment product (PT) present on the bundle of needles (14; 24), the bundle of needles being driven with reciprocating motion (T) along the longitudinal axis (X) during application.

8. The method as claimed in claim 7, wherein the method is a skin or lip tattoo removal method.

9. The method as claimed in claim 7, wherein the method is a permanent skin or lip makeup method.

10. The method as claimed in claim 7, wherein the method is a method of introducing hyaluronic acid into the skin or lips.

11. The method as claimed in claim 7, wherein the method is a method of removing the hyaluronic acid previously introduced into the skin or lips.
